(19) Europäisches Patentamt
European Patent Office
Office européen des brevets

(11) EP 1 054 001 B1

(12) EUROPEAN PATENT SPECIFICATION

(45) Date of publication and mention of the grant of the patent:
**09.07.2003 Bulletin 2003/28**

(51) Int Cl.⁷: **C07C 331/28**, C09K 19/18

(21) Application number: **99109835.1**

(22) Date of filing: **19.05.1999**

(54) **Isothiocyanate tolanes and liquid crystal mixtures containing them**

Isothioncyanat-Tolane und sie enthaltende flüssigkristalline Gemische

Tolanes d'isothiocyanate et mélanges de cristaux liquides les contenant

(84) Designated Contracting States:
**DE**

(43) Date of publication of application:
**22.11.2000 Bulletin 2000/47**

(73) Proprietors:
- **MERCK PATENT GmbH**
  **64293 Darmstadt (DE)**
- **Agency of Industrial Science and Technology**
  **Tokyo 100-8921 (JP)**
- **New Energy and Industrial Technology Development Organization**
  **Tokyo 170-6028 (JP)**

(72) Inventors:
- **Reiffenrath, Volker**
  **64380 Rossdorf (DE)**
- **Manabe, Atsutaka**
  **64625 Bensheim (DE)**

(56) References cited:
**WO-A-92/16519**
**DE-A- 4 301 700**
**DE-A- 4 445 224**
**GB-A- 2 290 787**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 054 001 B1

## Description

The invention relates to isothiocyanate tolanes and their use in liquid crystalline media and liquid crystal displays, and to liquid crystalline media and liquid crystal displays comprising such isothiocyanate tolanes.

For certain uses in the field of liquid crystal displays, for example in polymer dispersed liquid crystal displays based on the reflective scattering mode (RSM), it is required to have available liquid crystalline compounds and media with a very high value of the birefringence $\Delta n$. The current limit for birefringence of commercially used liquid crystal single compounds is around 0.32, whereas a reasonable minimum requirement for an effective RSM is 0.35. For specific uses, even liquid crystal materials with a $\Delta n$ value of more than 0.5 are required.

The main problems when using materials of the state of the art with high $\Delta n$ in practical applications are their UV instability and their poor solubility. A further drawback of these materials is their insufficient compatibility with the active matrix technology. Thus, known high $\Delta n$ materials often do exhibit a low dielectric anisotropy $\Delta\varepsilon$ and/or a low specific resistivity, which are serious disadvantages of these materials when used in active matrix applications.

It was therefore an aim of the present invention to provide novel liquid crystal materials with high $\Delta n$ for liquid crystal displays, in particular displays of the RSM or PDLC type, which exhibit broad mesophase ranges, high values of $\Delta n$, of $\Delta\varepsilon$ and of the specific resistivity, show satisfying solubility and stability, in particular UV stability, and do not show the disadvantages mentioned above.

The inventors have found that this aim can be achieved, and the above mentioned problems can be overcome, by providing novel isothiocyanate tolane derivatives according to the present invention. These compounds show highly promising properties and are very useful for applications in liquid crystal displays, especially in active matrix driven displays and/or displays of the RSM type.

WO 92/16519 discloses compounds of a broad generic formula comprising 2 or 3 phenylene rings which may, inter alia, also comprise -C≡C- bridging groups and terminal NCS groups. However, WO 92/16519 does not explicitly disclose isothiocyanate tolanes according to the present invention. Furthermore, the compounds explicitly disclosed in WO 92/16519 have only low values of $\Delta\varepsilon$ and further do only exhibit narrow smectic phases and at best monotropic nematic phases, or do not even exhibit nematic phases at all, which makes these compounds highly unsuitable for use in RSM or PDLC displays, in particular of the active matrix type.

One object of the present invention are isothiocyanate tolane derivatives of formula I

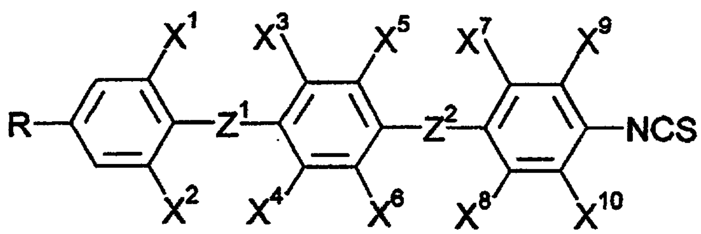 I

wherein

R is a straight-chain or branched alkyl group with 1 to 25 C atoms which may be unsubstituted, mono- or polysubstituted with halogen or CN, group with 1 to 25 C atoms which may be unsubstituted, mono- or polysubstituted by halogen or CN, it being also possible for one or more non-adjacent $CH_2$ groups to be replaced, in each case independently from one another, by -O-, -S-, -CO-, -COO-, -OCO-, -OCO-O-, -S-CO-, -CO-S-, -CH=CH-, -CH=CF-, -CF=CF- or -C≡C- in such a manner that oxygen atoms are not linked directly to one another,

one of $Z^1$ and $Z^2$ is -C≡C- and the other is -C≡C- or a single bond, and $X^1$ to $X^{10}$ are each independently H or F.

Another object of the present invention is the use of isothiocyanate tolane derivatives of formula I in liquid crystalline media and liquid crystal displays.

Another object of the present invention is a liquid crystalline medium comprising at least two components, at least one of which is an isothiocyanate tolane derivative of formula I.

Yet another object of the present invention is a liquid crystal display with a liquid crystalline medium comprising at least two components, at least one of which is an isothiocyanate tolane derivative of formula I.

Especially preferred are compounds of formula I, wherein one of $Z^1$ and $Z^2$ is -C≡C- and the other is a single bond.

Further preferred are compounds, wherein in at least one of the pairs of radicals $X^1$ and $X^2$, $X^3$ and $X^4$, $X^5$ and $X^6$, $X^7$ and $X^8$, $X^9$ and $X^{10}$ either both radicals denote F or both radicals denote H. Of these compounds, particularly

preferred are those wherein in at least one, preferably in one or two, of the pairs $X^1$ and $X^2$, $X^3$ and $X^4$, $X^5$ and $X^6$, $X^7$ and $X^8$, $X^9$ and $X^{10}$ both radicals are denoting F.

Further preferred are compounds wherein R is straight chain alkyl, alkoxy or alkenyl with 1 to 10 C-atoms.

Particularly preferred are compounds of the following formulae

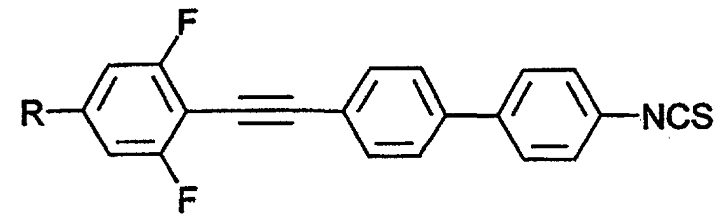

Ia

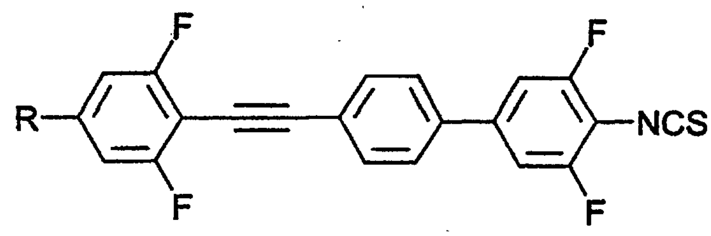

Ib

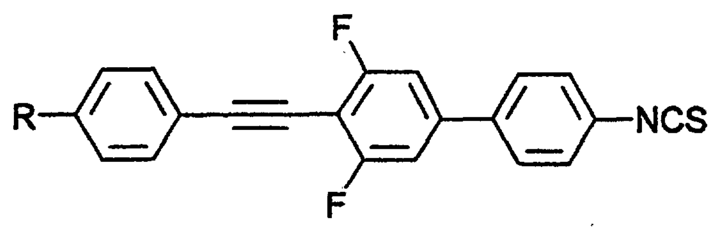

Ic

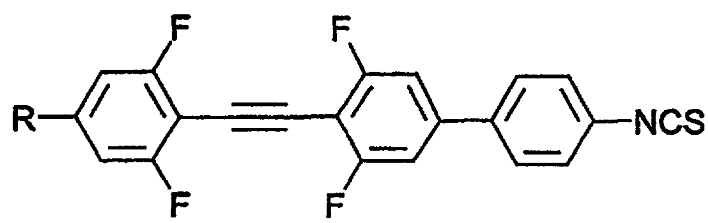

Id

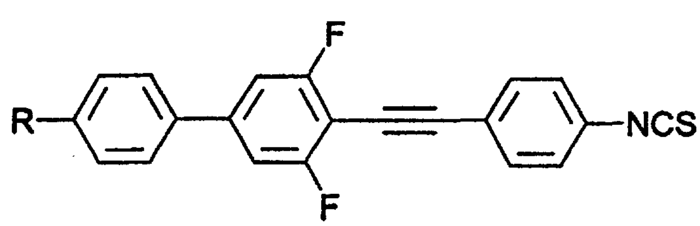

Ie

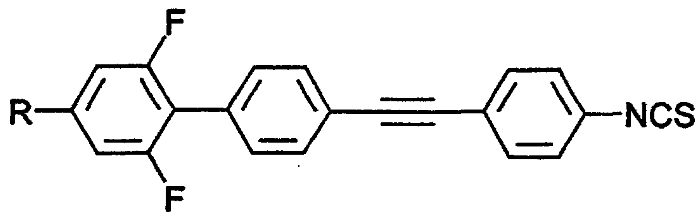

If

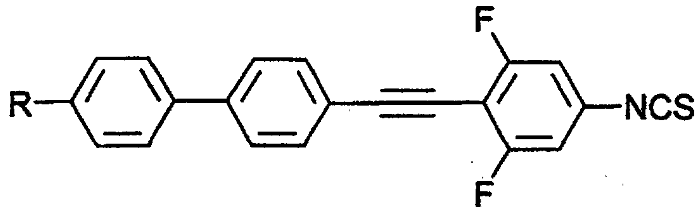

Ig

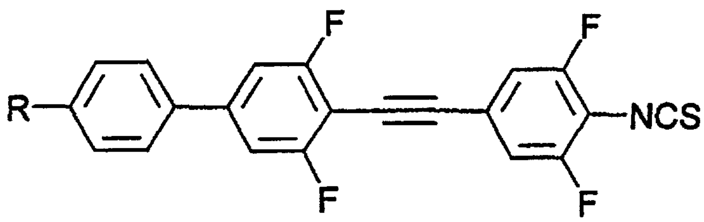

Ih

and compounds of the following formulae

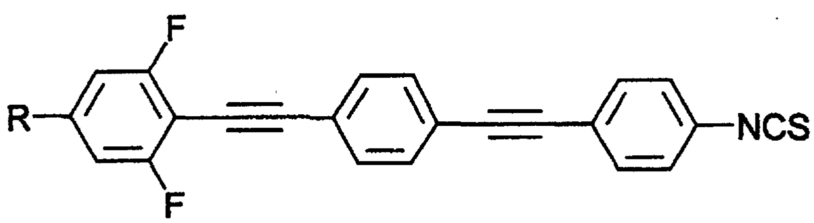

Ii

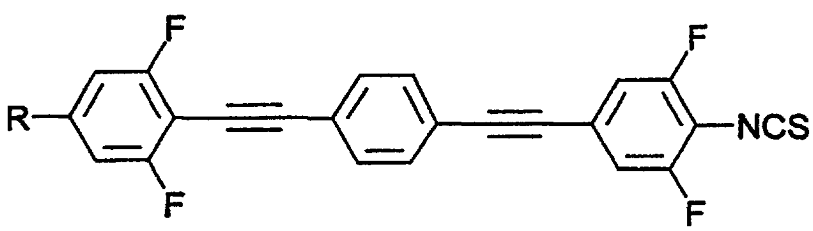

Ik

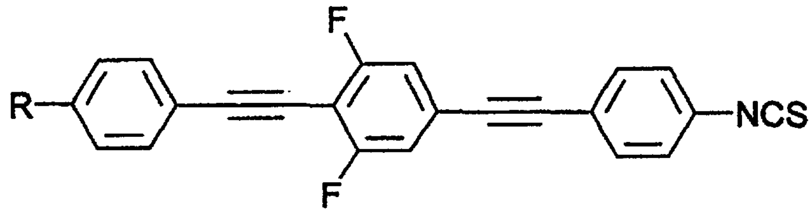

Im

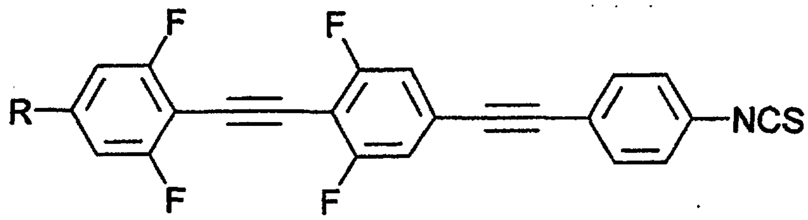

In

wherein R has the meaning of formula I.

Especially preferred are compounds of formula 1a to 1h, in particular compounds of formula 2a and 1b.

If R is an alkyl or alkoxy group, i.e. where one or more $CH_2$ groups are replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, methoxy, nonoxy, decoxy, undecoxy, dodecoxy, tridecoxy or tetradecoxy, for example.

Oxaalkyl, i.e. where one $CH_2$ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

If R is an alkenyl group, i.e. wherein one or more $CH_2$ groups are replaced by -CH=CH-, this may be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are $C_2$-$C_7$-1E-alkenyl, $C_4$-$C_7$-3E-alkenyl, $C_5$-$C_7$-4-alkenyl, $C_6$-$C_7$-5-alkenyl und $C_7$-6-alkenyl, in particular $C_2$-$C_7$-1E-alkenyl, $C_4$-$C_7$-3E-alkenyl and $C_5$-$C_7$-4-alkenyl. Examples for particularly preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C atoms are generally preferred.

If R is an alkyl or alkenyl group that is monosubstituted by CN, it is preferably straight-chain. The substitution by CN can be in any desired position.

If R is an alkyl or alkenyl group that is at least monosubstituted by halogen, it is preferably straight-chain. Halogen is preferably F or Cl, in case of multiple substitution preferably F. The resulting groups include also perfluorinated groups. In case of monosubstitution the F or Cl substituent can be in any desired position, but is preferably in $\omega$-position. Examples for especially preferred straight-chain groups with a terminal F substituent are fluormethyl, 2-fluorethyl, 3-fluorpropyl, 4-fluorbutyl, 5-fluorpentyl, 6-fluorhexyl and 7-fluorheptyl. Other positions of F are, however, not excluded.

Compounds of formula I with branched groups R can occasionally be of importance due to their better solubility in the conventional liquid crystalline media. Optically active compounds of this type are in particular useful as chiral dopants. Optically active and smectic compounds of formula I are useful as components for ferroelectric media and displays.

The inventive compounds of formula I can be synthesized according to or in analogy to methods known per se and described in the literature (for example in standard works of organic chemistry, such as Houben-Weyl, Methoden der organischen Chemie [Methods of Organic Chemistry], Georg-Thieme-Verlag, Stuttgart), to be precise under reaction conditions which are known and suitable for said reactions. Use can also be made here of variants which are known per se, but are not described here in greater detail.

Some specific methods of preparation can be taken from the examples.

Furthermore, the inventive compounds of formula I can be prepared according to or in analogy to reaction scheme 1.

## Scheme 1

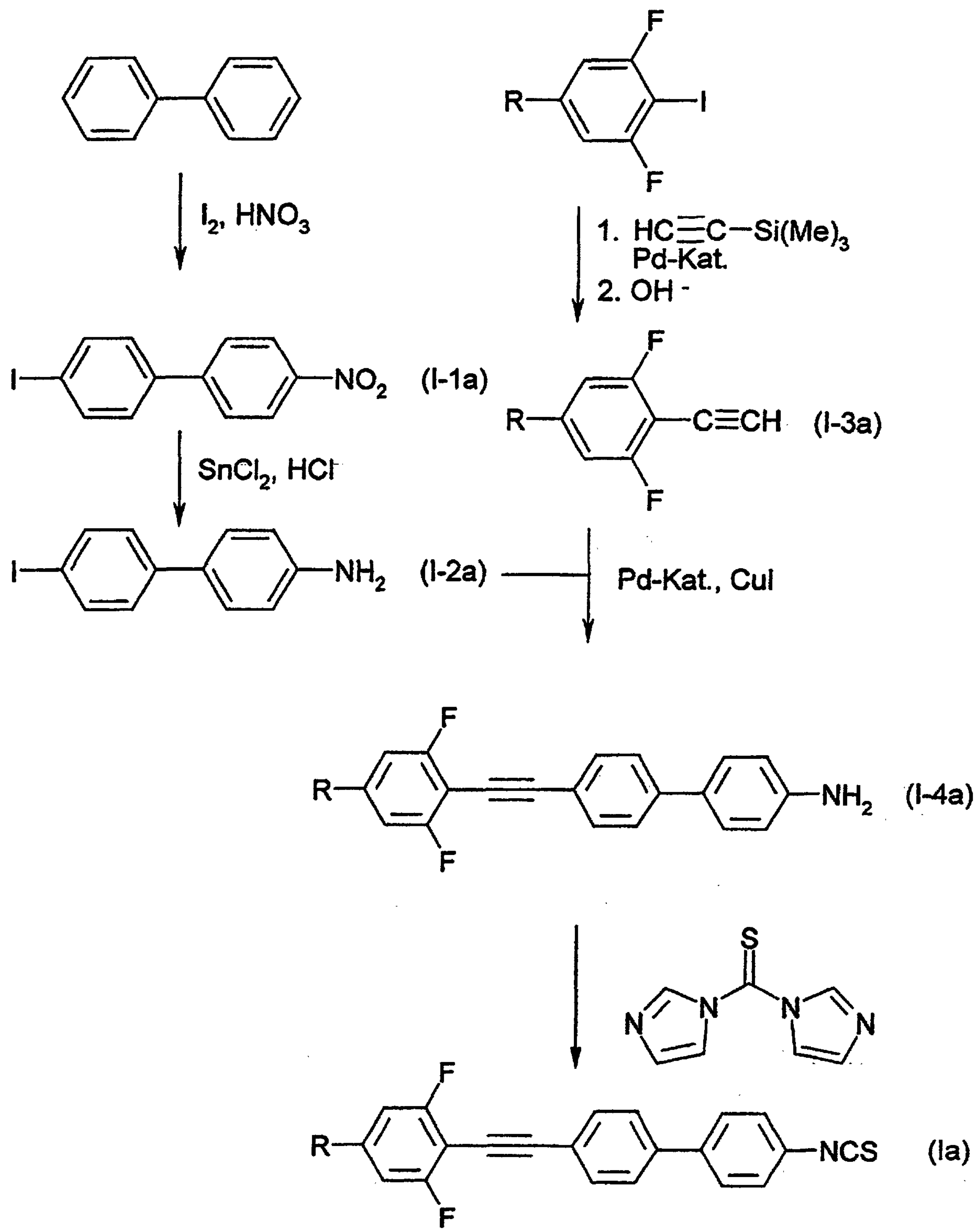


wherein R has the meaning of formula I.

The inventive compounds of formula I are particularly useful as components in liquid crystalline media.

Liquid crystalline media according to the invention preferably comprise 2 to 40, especially preferably 4 to 30 components as further constituents in addition to one or more inventive compounds. Very preferably these media comprise 2 to 25, preferably 3 to 15 compounds, at least one of which is a compound of formula I. The further constituents are preferably low molecular weight liquid crystalline compounds selected from nematic or nematogenic substances, for example from the known classes of the azoxy-benzenes, benzylidene-anilines, biphenyls, terphenyls, phenyl or cyclohexyl benzoates, phenyl or cyclohexyl esters of cyclohehexanecarboxylic acid, phenyl or cyclohexyl esters of cyclohexylbenzoic acid, phenyl or cyclohexyl esters of cyclohexylcyclohexane carboxylic acid, cyclohexylphenyl esters of benzoic acid, of cyclohexane carboxylic acid and of cyclohexylcyclohexane carboxylic acid, phenylcyclohexanes, cyclohexylbiphenyls, phenylcyclohexylcyclohexanes, cyclohexylcyclohexanes, cyclohexylcyclohexenes, cyclohexylcyclohexylcyclohexenes, 1,4-bis-cyclohexylbenzenes, 4,4'-bis-cyclohexylbiphenyls, phenyl- or cyclohexylpyrimidines, phenyl- or cyclohexylpyridines, phenyl- or cyclohexylpyridazines, phenyl- or cyclohexyldioxanes, phenyl- or cyclohexyl-1,3-dithianes, 1,2-di-phenylethanes, 1,2-dicyclohexylethanes, 1-phenyl-2-cyclohexylethanes, 1-cyclohexyl-2-(4-phenylcyclohexyl)ethanes, 1-cyclohexyl-2-biphenylethanes, 1-phenyl-2-cyclohexyl-phenylethanes, optionally halogenated stilbenes, benzyl phenyl ether, tolanes, substituted cinnamic acids and further classes of nematic or nematogenic substances. The 1,4-phenylene groups in these compounds may also be laterally mono- or difluorinated.

The preferred liquid crystalline media are based on the achiral compounds of this type.

The most important compounds suitbale as components of the inventive liquid crystalline media can be characterized by the following formulae 1 to 6

$$R'\text{-}L\text{-}E\text{-}R'' \qquad 1$$

$$R'\text{-}L\text{-}COO\text{-}E\text{-}R'' \qquad 2$$

$$R'\text{-}L\text{-}OOC\text{-}E\text{-}R'' \qquad 3$$

$$R'\text{-}L\text{-}CH_2CH_2\text{-}E\text{-}R'' \qquad 4$$

$$R'\text{-}L\text{-}C{\equiv}C\text{-}E\text{-}R'' \qquad 5$$

$$R'\text{-}L\text{-}CH{=}CH\text{-}E\text{-}R'' \qquad 6$$

wherein L' and E, which may be identical or different, are in each case, independently of one another, a bivalent radical from the group formed by -Phe-, -Cyc-, -Phe-Phe-, -Phe-Cyc-, -Cyc-Cyc-, -Pyr-, -Dio-, -G-Phe- and -G-Cyc- and their mirror images, where Phe is unsubstituted or fluorine-substituted 1,4-phenylene, Cyc is trans-1,4-cyclohexylene or 1,4-cyclohexenylene, Pyr is pyrimidine-2,5-diyl or pyridine-2,5-diyl, Dio is 1,3-dioxane-2,5-diyl and G is 2-(trans-1,4-cyclohexyl)ethyl, pyrimidine-2,5-diyl, pyridine-2,5-diyl or 1,3-dioxane-2,5-diyl.

In the case of compounds of formula 6, the rings L and E directly linked to the CH=CH group are preferably each Cyc.

One of the radicals L and E is preferably Cyc, Phe or Pyr. E is preferably Cyc, Phe or Phe-Cyc. The inventive media preferably comprise one or more components selected from the compounds of formulae 1 to 5 in which L and E are selected from the group consisting of Cyc, Phe and Pyr, and simultaneously one or more components selected from the compounds of formulae 1 to 5 in which one of the radicals L and E is selected from the group consisting of Cyc, Phe and Pyr and the other radical is selected from the group consisting of -Phe-Phe-, -Phe-Cyc-, -Cyc-Cyc-, -G-Phe-und -G-Cyc-, and optionally one or more components selected from the compounds of formulae 1 to 5 in which the radicals L and E are selected from the group consisting of -Phe-Cyc-, -Cyc-Cyc-, -G-Phe-and -G-Cyc-.

In a smaller subgroup of compounds of formulae 1, 2, 3, 4, 5 and 6 R' and R", in each case independently of one another, denote alkyl, alkenyl, alkoxy, alkoxyalkyl, alkenyloxy or alkanoyloxy having up to 8 C atoms. In the following this smaller subgroup is called group A, and the compounds forming this subgroup are labelled with the subformulae 1a, 2a, 3a, 4a, 5a and 6a. In most of these compounds R' and R" are different, and one of these radicals is usually alkyl, alkenyl, alkoxy or alkoxyalkyl.

In another smaller subgroup of compounds of formulae 1, 2, 3, 4, 5 and 6, which is called group B, R" is -F, -Cl, -NCS or $-(O)_iCH_{3-(k+l)}F_kCl_l$, with i being 0 or 1 and k+l being 1, 2 or 3; the compounds wherein R" has this meaning are labelled with the subformulae 1b, 2b, 3b, 4b, 5b and 6b. Especially preferred are compounds of subformulae 1b, 2b, 3b, 4b, 5b and 6b wherein R" is -F, -Cl, -NCS, $-CF_3$, $-OCHF_2$ or $-OCF_3$.

In the compounds of subformulae 1b, 2b, 3b, 4b, 5b and 6b R' is as defined given for the compounds of the subformulae 1a-6a, and is preferably alkyl, alkenyl, alkoxy or alkoxyalkyl.

In a further smaller subgroup of compounds of formulae 1, 2, 3, 4, 5 and 6 R" is -CN; this subgroup in the following is called group C and the compounds of this subgroup are labelled with the subformulae 1c, 2c, 3c, 4c, 5c and 6 c. In the compounds of the subformulae 1c, 2c, 3c, 4c, 5c and 6c R' is as defined for the compounds of the subformulae 1a-6a, and is preferably alkyl, alkoxy or alkenyl.

Apart of the preferred compounds of groups A, B and C, other compounds of formulae 1, 2, 3, 4, 5 and 6 having other variants of the proposed substituents are also customary. Many of these compounds or mixtures thereof are commercially available. All these compounds can be prepared according to or in analogy to methods known per se and described in the literature (for example in standard works of organic chemistry, such as Houben-Weyl, Methoden der organischen Chemie [Methods of Organic Chemistry], Georg-Thieme-Verlag, Stuttgart), to be precise under reaction conditions which are known and suitable for said reactions. Use can also be made here of variants which are known per se, but are not described here in greater detail.

The inventive media in addition to compounds of formula I preferably comprise one or more compounds selected from group A and/or group B and/or group C. The proportions by weight of the compounds from these groups in the inventive media are preferably

Group A: 0 to 90 %, preferably 20 to 90 %, in particular 30 to 90 %
Group B: 0 to 80 %, preferably 10 to 80 %, in particular 10 to 65 %
Group C: 0 to 80 %, preferably 5 to 80 %, in particular 5 to 50 %

with the sum of the proportions by weight of the compounds from group A and/or group B and/or group C present in the inventive media being preferably 5 % to 90 %, in particular 20 % to 90 %.

The inventive media preferably comprise 1 to 40 %, in particular 5 to 30 % of inventive compounds of formula I. Further preferred are media comprising more than 40 %, in particular 45 to 90 % of compounds of formula I. The media preferably comprise 1 to 5, in particular 1, 2 or 3 or compounds of formula I.

The preparation of the media that can be used according to the invention is carried out by conventional methods. Usually the desired amount of the components used in lower proportion is dissolved in the components forming the main part of the medium, preferably at elevated temperatures. It is also possible to mix solutions of the components in an organic solvent, like e.g. aceton, chloroform or methanol, and to remove the solvent after mixing, e.g. by destillation.

The liquid crystal media can also comprise other additives that are known to the expert and are described in the literature (for example in standard works, such as H.Kelker/R. Hatz, Handbook of Liquid Crystals, Verlag Chemie, Weinheim 1980). For example, it is possible to add 0-15 % of pleochroic dyes, substances to modify the dielectric anisotropy, viscosity and/or the orientation of the nematic phases, or chiral dopants.

The inventive compounds can be used in liquid crystal media and liquid crystal displays, such as STN, TN, AMD-TN, temperature compensation, guest-host and scattering type displays. They are particularly useful in high $\Delta n$ liquid crystal media and displays using such media, especially RSM and PDLC type displays.

In the foregoing and in the following examples, unless otherwise indicated, all temperatures are set forth uncorrected in degrees Celsius and all parts and percentages are by weight. The following abbreviations are used to illustrate the liquid crystalline phase behaviour of the compounds: K = crystalline; N = nematic; S = smectic; I = isotropic. The numbers between these symbols indicate the phase transition temperatures in degree Celsius. Furthermore, $\Delta n$ is the birefringence at 589 nm and 20 °C and $\Delta\varepsilon$ is the dielectric anisotropy at 20 °C.

Example 1

Compound (1) is prepared according to reaction scheme 1

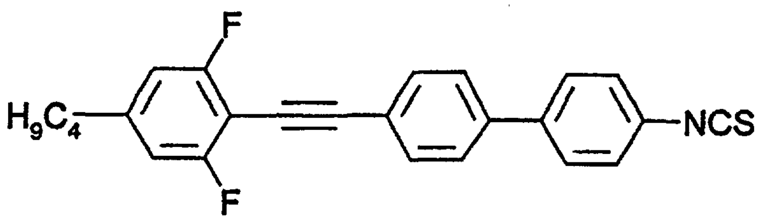

(1)

and has the following properties

$$K\ 102\ S_A\ 208\ N\ 259.8\ I,\ \Delta\varepsilon = +17.8,\ \Delta n = 0.497$$

($\Delta n$ and $\Delta\varepsilon$ have been extrapolated from a solution of 5 % in the commercially available nematic host mixture ZLI-4792 (from Merck KGaA, Darmstadt, Germany) having the properties K < - 40 N 91 I, $\Delta\varepsilon$ = + 5.2, $\Delta n$ = 0.094 and a translational viscosity of 15 $mm^2$/sec. at 20 °C).

<u>I-1a1) 4-Iodo-4'-nitro-biphenyl</u>

Biphenyl (150 g, 0.973 mol) was heated together with iodine (75 g, 0.295 mol) to 70 °C. $HNO_3$ (65 %, 360 ml, 7.999 mol) was added dropwise within 1h under vigorous gas formation. 20 ml dichloromethane were added, and the mixture was stirred under reflux at 75 °C for 1 h. Water was added to the mixture and the yellow precipitate separated, washed with water (5 x 500 ml) and separated again. The precipitate was suspended in toluene (500 ml), filtrated and washed with toluene (250 ml).

<u>I-2a1) 4'-Iodo-biphenyl-4-ylamine</u>

$SnCl_2$ dihydrate (350 g, 1.551 mol) was dissolved in HCl (37 %, 500 ml). The solution was heated to 50 °C and 4-iodo-4'-nitro-biphenyl (I-1a1) (97 g, 0.283 mol) was added. The suspension thus obtained was stirred overnight at 60 °C. The mixture was allowed to cool to room temperature, cast onto ice/water, and NaOH was added (1300 ml, conc.). The mixture was extracted with toluene (3000 ml) and the phases separated. The organic phase was washed with water (2 x 1000 ml), the aqueous phases were extracted with toluene (1000 ml). The organic phases were dried over $Na_2SO_4$, filtrated and evaporated to dryness.

<u>(2,6-Difluoro-4-butyl-phenylethynyl)-trimethyl-silane</u>

1,3-Difluoro-2-iodo-5-butyl benzene (15.015 g, 0.05 mol) and trimethylsilyl acetylene (9.9 ml, 0.07 mol) are dissolved at room temperature in triethylamine (100 ml). Bis-triphenylphosphine palladium (0.702 g, 1 mol) and copper (I)iodide (0.095 g, 0.5 mol) are added and the mixture stirred overnight at room temperature. Water and methyl tert. butyl (MTB) ether were added to the mixture, followed by filtration over celite, and the phases separated. The organic phase was washed two times with ammonium chloride solution, and the aqueous phases extracted with MTB ether three times. The organic phases were dried over $Na_2SO_4$, filtrated and the solvent partially removed.

<u>I-3a1) 2-Ethynyl-1,3-difluoro-5-butyl-benzene</u>

KOH (3.254 g, 0.057 mol) was dissolved in methanol (120 ml), ) 2,6-difluoro-4-butyl-phenylethynyl)-trimethyl-silane (13.8 g, 0.052 mol) were added and the yellowish brown solution stirred overnight at room temperature. The mixture was cast onto ice/conc. HCl, extracted with hexane and the phases separated. The organic phase was washed with water two times and the aqueous phases extracted with hexane three times. The organic phases were dried over $Na_2SO_4$, filtrated and the solvent partially removed.

<u>I-4a1) 4' -(2,6-Difluoro-4-butyl-phenylethynyl)-biphenyl-4-ylamine</u>

2-Ethynyl-1,3-difluoro-5-butyl-benzene (I-3a1) (8.7 g, 0.043 mol) and 4'-Iodo-biphenyl-4-ylamine (I-2a1) (13.779 g, 0.043 mol) were suspended in triethylamine (150 ml). Bis-triphenylphosphine palladium (0.597 g, 0.85 mol)

and copper(I)iodide (0.081 g, 0.425 mol) were added and the mixture stirred overnight at room temperature. Water was added to the mixture, and the mixture extracted with MTB ether and the phases separated. The organic phase was washed with ammonium chloride solution three times and the aqueous phases extracted with MTB ether three times. The organic phases were dried over $Na_2SO_4$, filtrated and the solvent partially removed.

Ia1) 4 -(2,6-Difluoro-4-butyl-phenylethynyl)-4'-isothiocyanato- biphenyl

4'-(2,6-Difluoro-4-butyl-phenylethynyl)-biphenyl-4-ylamine (I-4a1) (12.2 g, 0.034 mol) and 1,1-Thiocarbonyldiimidazole (12.119 g, 0.068 mol) were dissolved in dichloromethane (100 ml) and stirred overnight at room temperature. The solvent was evaporated off and the crude product was eluated with 1-chlorobutane over silica gel. The product was purified by repeated recrystallization from n-hexane at 20 °C, with the last recrystallization step carried out over activated carbon and celite.

## Claims

**1.** Isothiocyanate tolane derivatives of formula I

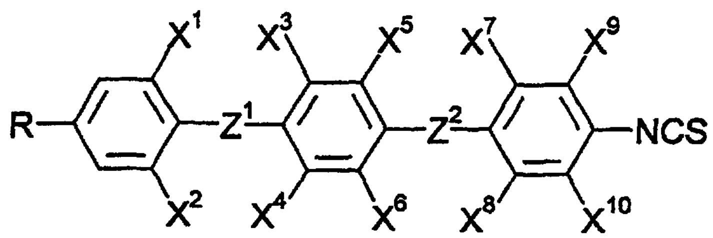

I

wherein

R is a straight-chain or branched alkyl group with 1 to 25 C atoms which may be unsubstituted, mono- or polysubstituted by halogen or CN, it being also possible for one or more non-adjacent $CH_2$ groups to be replaced, in each case independently from one another, by -O-, -S-, -CO-, -COO-, -OCO-, -OCO-O-, -S-CO-, -CO-S-, -CH=CH-, -CH=CF-, -CF=CF- or -C≡C- in such a manner that oxygen atoms are not linked directly to one another,

one of $Z^1$ and $Z^2$ is -C≡C- and the other is -C=C- or a single bond,
and
X' to $X^{10}$ are each independently H or F.

**2.** Isothiocyanate tolane derivatives according to claim 1, wherein one of $Z^1$ and $Z^2$ is -C≡C- and the other is a single bond.

**3.** Isothiocyanate tolane derivatives according to claim 1 or 2, wherein in at least one of the pairs of radicals $X^1$ and $X^2$, $X^3$ and $X^4$, $X^5$ and $X^6$, $X^7$ and $X^8$, $X^9$ and $X^{10}$ both radicals denote F.

**4.** Isothiocyanate tolane derivatives according to at least one of claims 1 to 3, selected of the following formulae

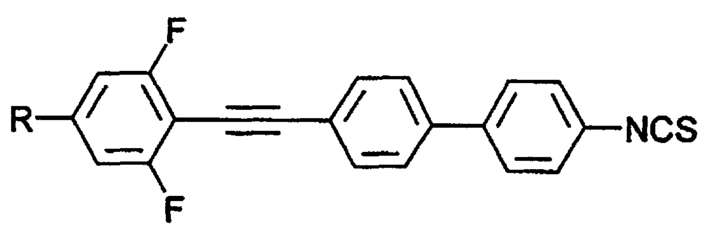

Ia

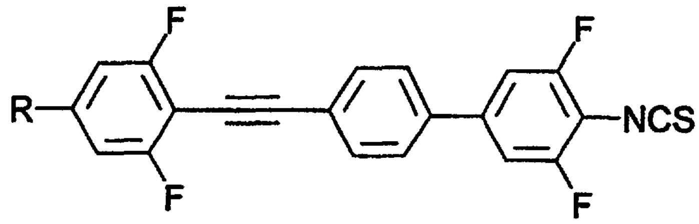

Ib

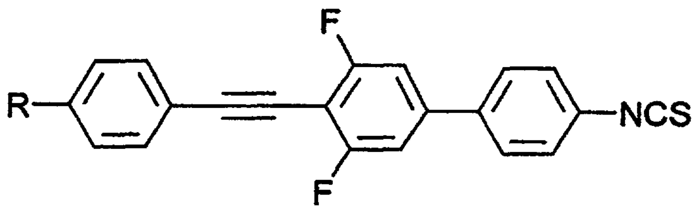

Ic

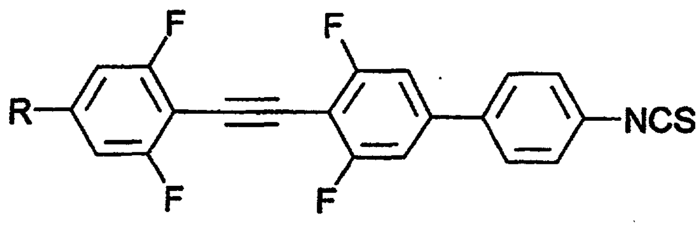

Id

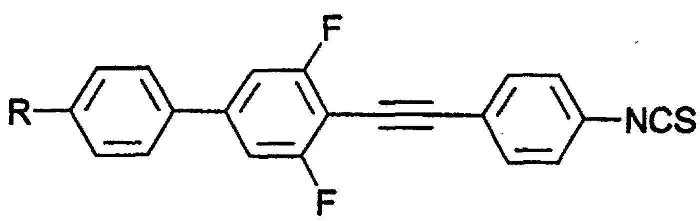

Ie

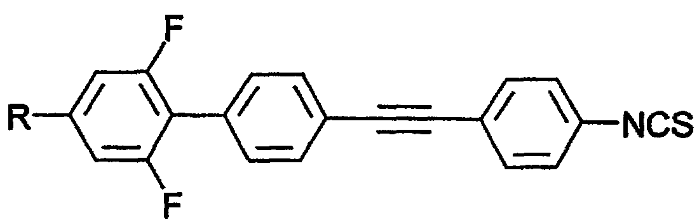

If

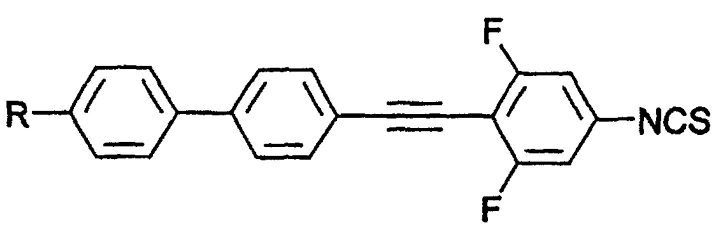

Ig

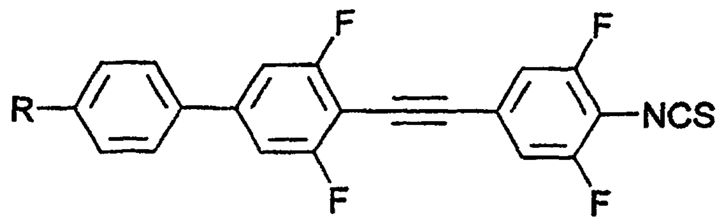

Ih

wherein R has one of the meanings of formula I.

5. Isothiocyanate tolane derivatives according to at least one of claims 1 to 4, wherein R is straight chain alkyl, alkoxy or alkenyl with 1 to 10 C-atoms.

6. Use of isothiocyanate tolane derivatives according to at least one of claims 1 to 5 in liquid crystalline media and liquid crystal displays.

7. Liquid crystalline medium comprising at least two components, at least one of which is an isothiocyanate tolane derivative according to at least one of claims 1 to 5.

8. Liquid crystal display with a liquid crystalline medium comprising at least two components, at least one of which is an isothiocyanate tolane derivative according to at least one of claims 1 to 5.

**Patentansprüche**

1. Isothiocyanatotolanderivate der Formel I

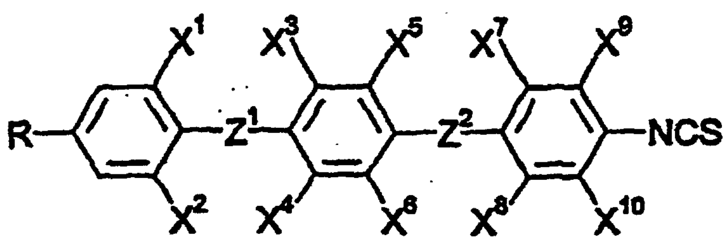

I

worin

R eine geradkettige oder verzweigte Alkylgruppe mit 1 bis 25 C- Atomen, die unsubstituiert oder ein- oder mehrfach mit Halogen oder CN substituiert sein kann, wobei auch eine oder mehrere nicht benachbarte $CH_2$-Gruppen jeweils unabhängig voneinander durch -O-, -S-, -CO-, -COO-, -OCO-, -OCO-O-, -S-CO-, -CO-S-, -CH=CH-, -CH=CF-, -CF=CF- oder -C≡C- so ersetzt sein können, dass Sauerstoffatome nicht direkt miteinander verknüpft sind,

eines von $Z^1$ und $Z^2$ -C≡C- und das andere -C≡C- oder eine Einfachbindung
und
$X^1$ bis $X^{10}$ jeweils unabhängig voneinander H oder F
bedeuten.

2. Isothiocyanatotolanderivate nach Anspruch 1, worin eines von $Z^1$ und $Z^2$ -C≡C- und das andere eine Einfachbindung bedeutet.

3. Isothiocyanatotolanderivate nach Anspruch 1 oder 2, worin in mindestens einem der Restepaare $X^1$ und $X^2$, $X^3$ und $X^4$, $X^5$ und $X^6$, $X^7$ und $X^8$, $X^9$ und $X^{10}$ beide Reste F bedeuten.

**4.** Isothiocyanatotolanderivate nach mindestens einem der Ansprüche 1 bis 3, die aus den folgenden Formeln ausgewählt sind

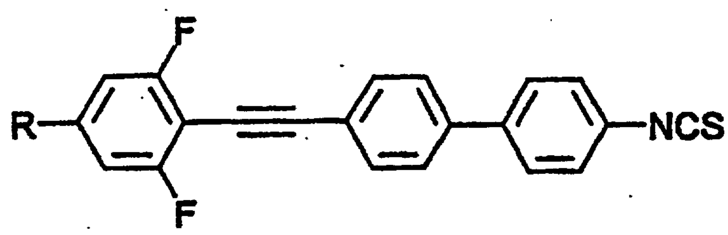

Ia

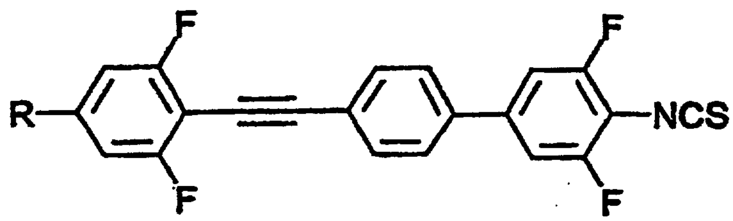

Ib

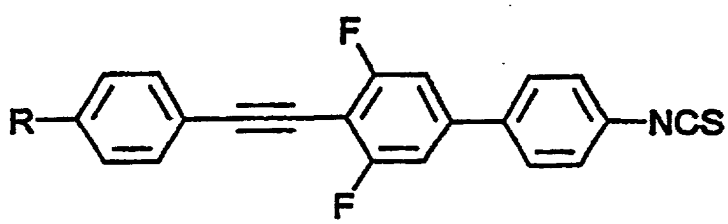

Ic

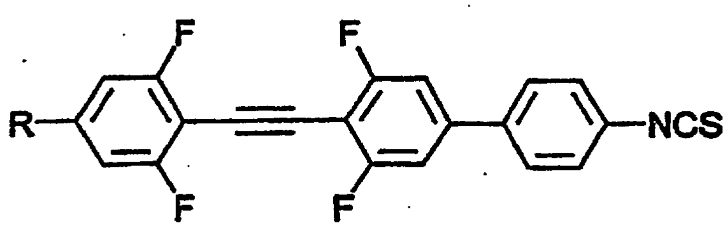

Id

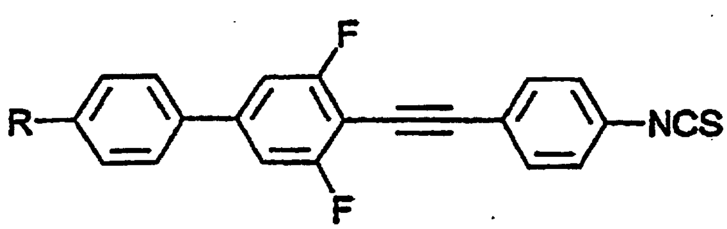

Ie

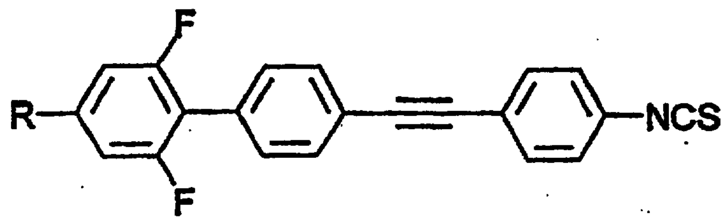

If

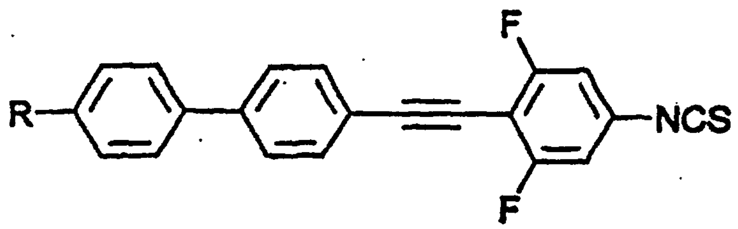

Ig

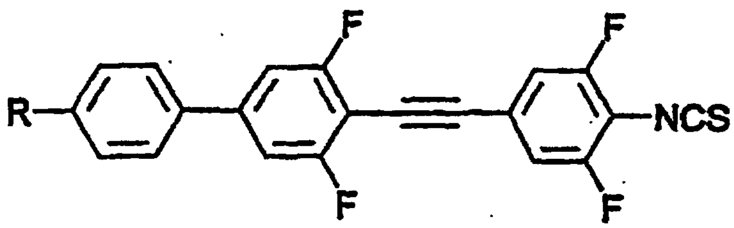

Ih

worin R eine der bei Formel I angegebene Bedeutungen hat.

5. Isothiocyanatotolanderivate nach mindestens einem der Ansprüche 1 bis 4, worin R ein geradkettiges Alkyl, Alkoxy oder Alkenyl mit 1 bis 10 C-Atomen ist.

6. Verwendung von Isothiocyanatotolanderivaten nach mindestens einem der Ansprüche 1 bis 5 in flüssigkristallinen Medien und Flüssigkristallanzeigen.

7. Flüssigkristallines Medium enthaltend mindestens zwei Komponenten, von denen mindestens eine ein Isothiocyanatotolanderivat nach mindestens einem der Ansprüche 1 bis 5 ist.

8. Flüssigkristallanzeige mit einem flüssigkristallinen Medium enthaltend mindestens zwei Komponenten, von denen mindestens eine ein Isothiocyanatotolanderivat nach mindestens einem der Ansprüche 1 bis 5 ist.

## Revendications

1. Dérivés de tolane d'isothiocyanate de formule I:

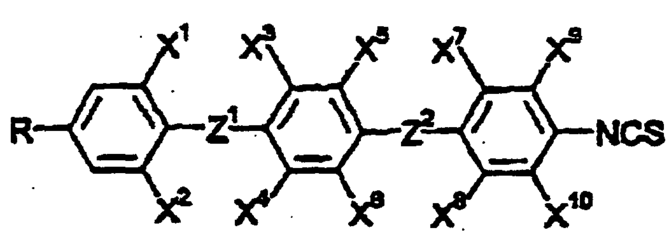

I

dans laquelle

R est un groupe alkyle à chaîne droite ou ramifiée avec de 1 à 25 atomes de carbone qui peuvent être non substitués, mono- ou poly substitués par un halogène ou CN, et il est également possible qu'un ou plusieurs groupes $CH_2$ non adjacents soient remplacés, dans chaque cas de manière indépendante l'un de l'autre, par - O -, - S -, - CO -, - COO -, - OCO -, - OCO - O -, - S -CO -, - CO - S -, - CH = CH -, - CH = CF -, - CF = CF - , ou - C≡C - , d'une manière telle que les atomes d'oxygène ne soient pas liés directement l'un à l'autre,

l'un parmi $Z^1$ et $Z^2$ est - C≡C - et l'autre est - C = C - ou une liaison simple,

et

$X^1$ à $X^{10}$ sons chacun indépendamment H ou F.

**2.** Dérivés de tolane d'isothiocyanate selon la revendication 1, dans laquelle l'un parmi $Z^1$ et $Z^2$ est - C ≡ C - et l'autre est une liaison simple

**3.** Dérivés de tolane d'isothiocyanate selon la revendication 1 ou 2, dans laquelle dans au moins l'une des paires de radicaux $X^1$ et $X^2$, $X^3$ et $X^4$, $X^5$ et $X^6$, $X^7$ et $X^8$, $X^9$ et $X^{10}$ les deux radicaux indiquent F.

**4.** Dérivés de tolane d'isothiocyanate selon au moins l'une des revendications 1 à 3, sélectionnés à partir des formules suivantes :

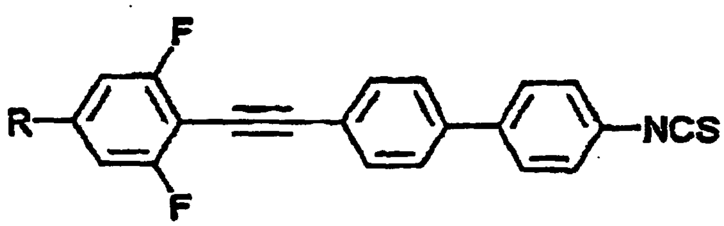

Ia

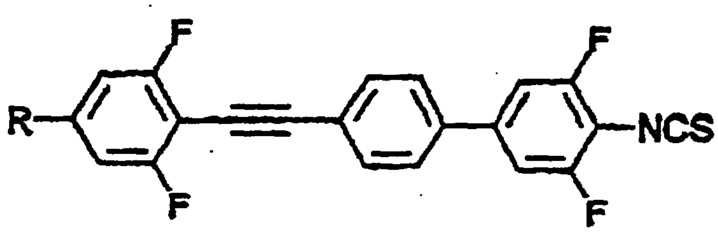

Ib

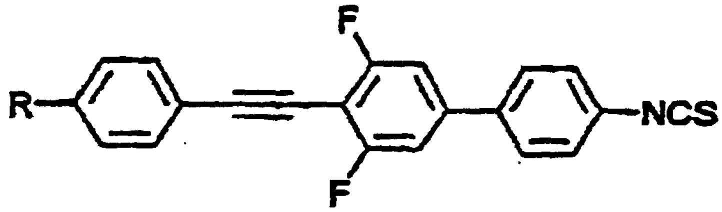

Ic

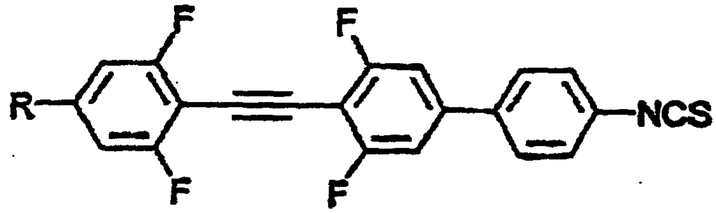

Id

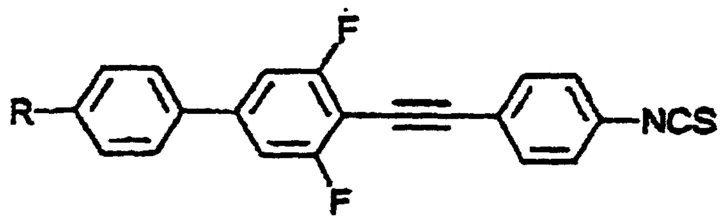

Ie

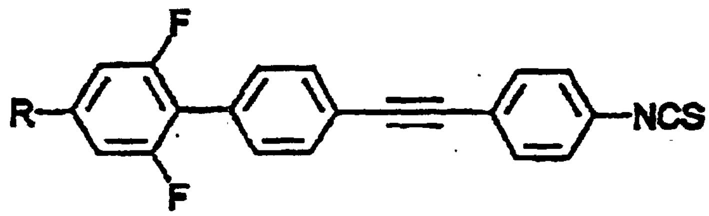

If

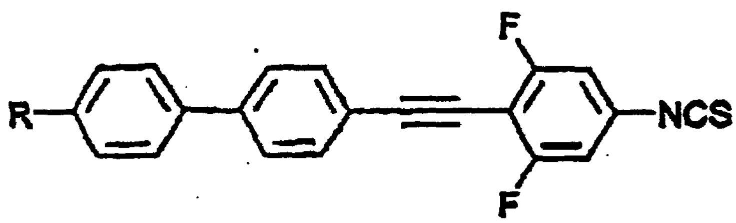

Ig

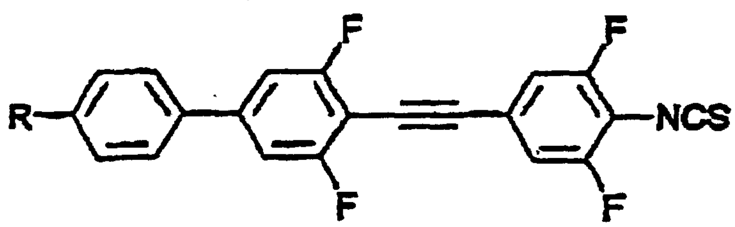

Ih

dans lesquelles R a l'une des significations de la formule L

**5.** Dérivés de tolane d'isothiocyanate selon au moins l'une des revendications 1 à 4, dans laquelle R est un alkyle, un alkoxy ou un alkényle à chaîne droite avec de 1 à 10 atomes de carbone.

**6.** Utilisation de dérivés de tolane d'isothiocyanate selon au mois l'une des revendications 1 à 5 dans des milieux cristallins et dans des écrans à cristaux liquides.

**7.** Milieu liquide cristallin comprenant au moins deux composants, l'un d'entre eux étant un dérivé de tolane d'isothiocyanate selon au moins l'une des revendications 1 à 5.

**8.** Ecran à cristaux liquides avec un milieu cristallin comprenant au moins deux composants, au moins l'un d'entre eux étant un dérivé de tolane d'isothiocyanate selon au moins l'une des revendications 1 à 5.